# FASCICULE DE BREVET EUROPEEN

(11) **EP 1 408 886 B1**
(45) Date de publication et mention de la délivrance du brevet: **07.03.2007**
(21) Numéro de dépôt: 02770052.5
(22) Date de dépôt: 22.07.2002
(51) Int. Cl.: A61F 2/34

(54) **COTYLE PROTHETIQUE MODULAIRE**
MODULARE PROTHETISCHE GELENKPFANNE
MODULAR ACETABULAR CUP

(30) Priorité: 23.07.2001 FR 0109796
(43) Date de publication de la demande: 21.04.2004
(73) Titulaire: Macara, Frédérique, 34130 Mauguio (FR)
(72) Inventeur: Macara, Frédérique, 34130 Mauguio (FR)
(74) Mandataire: Rhein, Alain
(86) Numéro de dépôt international: PCT/FR2002/002622
(87) Numéro de publication internationale: WO 2003/013397

(56) Documents cités:
- EP-A- 0 314 951
- EP-A- 0 551 794
- EP-A- 0 591 594
- EP-A- 1 082 949
- EP-A- 1 099 426
- WO-A-99/44545
- DE-A- 19 952 550
- FR-A- 2 793 137

## Description

La présente invention concerne un cotyle prothétique modulaire pour le traitement chirurgical de la reconstruction de l'articulation de la hanche dans les cas de reprises totales de prothèses de hanche, accompagnées ou non de perte de substance osseuse, et dans les cas de dysplasies d'autre part.

Le but du cotyle objet de la présente invention consiste à restituer à l'articulation l'ensemble des critères biomécaniques en traitant individuellement, grâce à sa modularité, les éléments fondamentaux que sont la fixation et l'orientation de l'implant.

Ce principe permet de répondre aux besoins de chaque situation rencontrée et de s'adapter à ses exigences, autorisant ainsi la mise en charge rapide du patient.

La technique actuelle du traitement chirurgical des reconstructions cotyloïdiennes met en évidence une insuffisance tant au niveau de l'ancrage intra osseux des dispositifs prothétiques existants que de leur positionnement biomécanique.

Un tel dispositif prothétique est décrit dans le document FR 2 793 137, il comprend un cotyle métallique destiné à être fixé dans la cavité acétabulaire, et apte à loger un dispositif d'adaptation métallique recevant un insert en matière plastique, lui-même comprenant une cavité en forme de portion de sphère apte à recevoir la tête sphérique d'un élément fémoral prothétique.

L'ancrage limité en fixation primaire peut avoir, dans le temps, une incidence néfaste sur l'intégration de l'implant, en regard des forces de pression et de torsion qui lui sont imposées, pouvant provoquer un descellement et/ou une rupture du matériel posé.

Le positionnement de l'implant, quant à lui, souvent tributaire de l'état du cotyle osseux à traiter, ne permet pas une libre orientation dans tous les plans qui permettrait de restituer une biomécanique normale à l'articulation, ce qui peut ainsi porter préjudice à la stabilité de l'articulation entraînant bien souvent des luxations et/ou descellement de l'implant.

Le cotyle prothétique modulaire objet de la présente invention permet de remédier à ces insuffisances en apportant une réponse globale au traitement chirurgical de la reconstruction de l'articulation de la hanche, en considérant ses exigences mécaniques, anatomiques et biologiques.

Le cotyle prothétique modulaire objet de la présente invention comporte d'une part une platine-support destinée à être disposée dans la cavité acétabulaire et ancrée au moyen de vis d'ancrage, et d'autre part une cupule métallique destinée à être assemblée à ladite platine support, et il se caractérise essentiellement en ce qu'il comprend une entretoise orientable verrouillée sur ladite platine-support par un système d'assemblage de type à cônes morses; tandis que ladite cupule métallique est assemblée à ladite entretoise par un système d'assemblage de type à cônes morses.

Selon une caractéristique additionnelle du cotyle prothétique selon l'invention, la platine-support comporte au moins une patte d'appui interne acétabulaire munie d'oeillets permettant un ancrage intra-acétabulaire de ladite platine-support au moyen des vis d'ancrage.

Selon une autre caractéristique additionnelle du cotyle prothétique selon l'invention, la platine-support comporte au moins une patte externe sus-acétabulaire munie d'oeillets destinés à recevoir des vis de fixation de type spongieux ou cortical, ainsi qu'un crochet obturateur apte à assurer un crochetage sous-acétabulaire.

La multiplication des points d'ancrage et d'appui de la platine-support, en zone périphérique grâce aux pattes sus-acétabulaires et au crochet obturateur, et en zone interne grâce aux pattes acétabulaires, permet d'atteindre une stabilité maximale de ladite platine-support et donc de l'implant.

On notera que dans les cas d'une grande destruction osseuse, les pattes de fixation sus-acétabulaires peuvent également être fixées au moyen de vis d'ancrage identiques à celles servant à la fixation des pattes acétabulaires.

Cette association des vis d'ancrage et de la platine-support représente une véritable ostéosynthèse et sa stabilité mécanique primaire demeure un élément essentiel de la pérennité de la reconstruction, et autorise la mise en charge rapide du patient.

Selon une autre caractéristique additionnelle du cotyle prothétique selon l'invention, il comporte un revêtement ostéoconducteur bicouche titane et hydroxyapatite.

La fixation secondaire de ce cotyle prothétique est optimisée d'une part par le remplissage de la cavité acétabulaire de greffes et/ou substituts osseux, participant à la reconstruction du stock osseux détruit, et d'autre part grâce au double revêtement ostéoconducteur qui peut être appliqué essentiellement à l'arrière de la ou les pattes acétabulaires de la platine-support, ainsi qu'à l'arrière de la cupule métallique qui vient se loger dans ladite platine-support par l'intermédiaire de l'entretoise.

Selon une autre caractéristique additionnelle du cotyle prothétique selon l'invention, l'entretoise comporte d'une part un cône mâle situé à sa base et venant se verrouiller sur un cône femelle que comporte la platine-support; et d'autre part un cône femelle situé sur sa partie supérieure, qui lui permet d'accueillir le cône mâle que comporte la cupule, ce qui autorise l'orientation de cette dernière selon les besoins, quelle que soit la position de ladite platine-support.

Ainsi, l'entretoise est prévue orientable dans tous les plans en sorte de jouer un rôle d'interface entre la platine-support et la cupule, et de permettre de recentrer le point d'appui vertical anatomique à l'intérieur de ladite cupule et d'augmenter ainsi la congruence et la taille de la surface portante.

Indépendamment de la fixation de la platine-support dans le cotyle osseux, le réglage de l'orientation anatomique du cotyle prothétique est assuré par la cupule métallique qui est orientable à 180° sur le plan vertical, antéropostérieur, et par l'entretoise orientable sur les plans vertical et horizontal, qui vient s'interposer entre la platine-support et la cupule métallique.

Ces deux éléments permettent le réglage très fin des axes anatomiques de l'articulation.

L'assemblage de type à cônes morses des différents éléments qui composent le cotyle prothétique selon l'invention assure le verrouillage de l'ensemble et fiabilise sa stabilité sur le long terme.

Selon une autre caractéristique additionnelle du cotyle prothétique selon l'invention, l'entretoise est pourvue, sur sa partie extérieure, au centre de sa partie la plus large, et sur plus de la moitié de sa circonférence, d'une collerette de verrouillage comprenant en divers points des oeillets permettant le passage de vis destinées à être serrées dans des trous taraudés pratiqués dans un épaulement périphérique que comporte la platine-support, ladite collerette présentant au niveau de chacun desdits oeillets un profil concave apte, en coopération avec lesdites vis dont les têtes présentent une surface d'appui de profil hémisphérique, à permettre une orientation desdites vis selon plusieurs axes.

Selon une variante, la cupule est munie sur sa zone équatoriale externe, sur plus de la moitié de sa circonférence, et au-dessus de son cône mâle, d'une collerette apte à permettre un verrouillage supplémentaire de la platine, de l'entretoise et de la cupule, à cet effet ladite collerette comprend en divers points des oeillets permettant le passage de vis destinées à être serrées dans des trous taraudés pratiqués dans un épaulement périphérique que comporte la platine-support, ladite collerette présentant au niveau de chacun desdits oeillets un profil concave apte, en coopération avec lesdites vis dont les têtes présentent une surface d'appui de profil hémisphérique, à permettre une orientation desdites vis selon plusieurs axes.

Selon un premier mode de réalisation particulier du cotyle prothétique selon l'invention, la cupule présente une partie interne totalement borgne, de type miroir, qui permet la libre mobilité d'un insert polymère qu'elle y accueille, lequel insert présente une cavité hémisphérique destinée à recevoir la tête sphérique d'un élément fémoral prothétique.

Cette architecture favorise l'amplitude de mouvement de l'articulation.

Selon un second mode de réalisation particulier du cotyle prothétique selon l'invention, la cupule présente une partie interne totalement borgne présentant une partie femelle et destinée à accueillir un insert céramique présentant un profil conique mâle en vue de réaliser un assemblage de type à cônes morses, ledit insert comportant une cavité hémisphérique destinée à recevoir la tête sphérique d'un élément fémoral prothétique.

Cette architecture, où l'insert est fixe par rapport à la cupule, répond à des prescriptions spécifiques, pour des patients jeunes et/ou très actifs par exemple.

Ces différentes caractéristiques, du cotyle prothétique objet de l'invention garantissent la restitution d'une biomécanique normale, gage de pérennité de la nouvelle prothèse, et permettent une rééducation et la reprise à une vie active plus rapides pour le patient.

Selon une autre caractéristique additionnelle du cotyle prothétique, selon l'invention, les vis d'ancrage comportent des moyens aptes à assurer le réglage affiné de la hauteur des surfaces d'appui dudit implant, et l'ancrage de ce dernier dans le tissu osseux tout en assurant un réglage équilibré des forces de pression.

Ainsi, le cotyle prothétique selon l'invention permet de traiter individuellement d'une part la fixation primaire et secondaire du fait notamment des caractéristiques des vis d'ancrage, et d'autre part l'orientation anatomique de l'implant du fait de sa large modularité et dont la stabilité est fiabilisée par le système d'assemblage de type à cônes morses de chaque élément constitutif du cotyle prothétique.

En effet, la fixation primaire de ce cotyle prothétique est assurée par la platine-support, par l'intermédiaire des vis d'ancrage qui permettent un ancrage en zone osseuse saine en exploitant tous les appuis disponibles, sans contrainte d'orientation.

Selon une autre caractéristique additionnelle du cotyle prothétique selon l'invention, les vis d'ancrage comportent chacune un corps comprenant une partie proximale de forme cylindrique prolongée d'une partie distale terminée en pointe et présentant un filetage de type spongieux auto taraudeur, ladite partie proximale étant percée axialement d'un canal taraudé destiné à accueillir un piston fileté, lui-même muni d'une tête et percé d'un puits taraudé destiné à recevoir une contre-vis de verrouillage.

A la manière d'un vérin mécanique, le piston fileté permet selon sa profondeur d'enfoncement dans le canal taraudé, de régler la hauteur des surfaces d'appui de l'implant prothétique, tandis que la contre-vis assure le serrage dudit implant.

Les avantages et les caractéristiques du dispositif selon l'invention, ressortiront plus clairement de la description qui suit et qui se rapporte au dessin annexé, lequel en représente plusieurs modes de réalisation non limitatifs.

Dans le dessin annexé :
- la figure 1 représente une vue schématique partielle et en éclaté d'un cotyle prothétique selon l'invention.
- la figure 2 représente une vue schématique partielle en coupe médiane d'une variante du même cotyle prothétique.
- la figure 3 représente une vue en perspective d'une variante d'une partie du cotyle de la figure 2.
- la figure 4 représente une vue en perspective d'une variante d'une autre partie du même cotyle.
- la figure 5 représente une vue en perspective d'une variante d'une autre partie du même cotyle
- la figure 6 représente une vue schématique en coupe partielle selon un plan longitudinal, et en éclaté, d'une vis d'ancrage pour la fixation du cotyle selon l'invention.

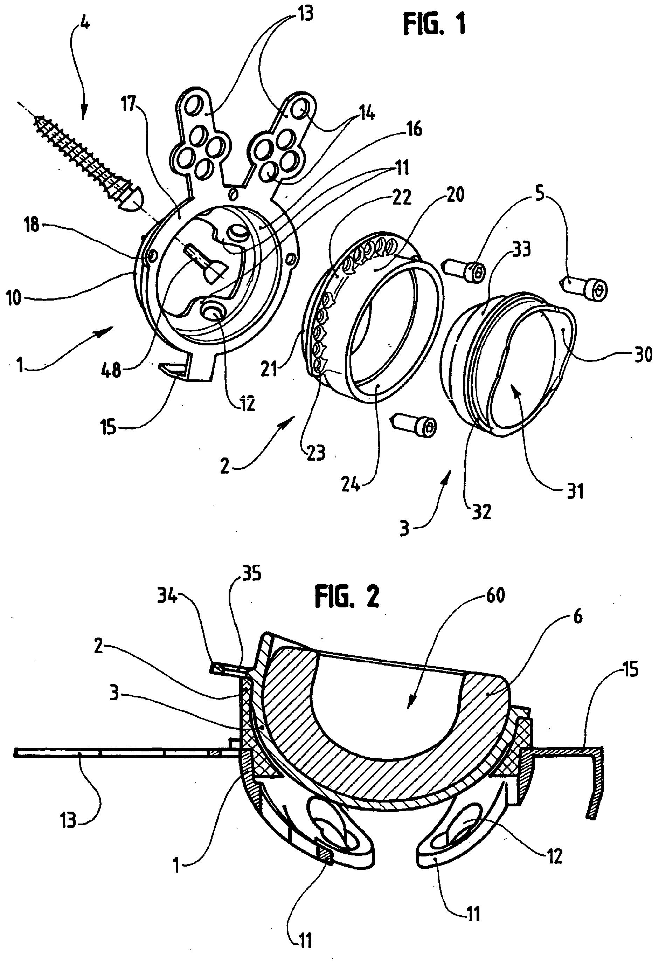

En référence à la figure 1, on peut voir un cotyle prothétique selon l'invention. Il comporte une platine-support 1 destinée à être implantée dans la cavité acétabulaire, et à recevoir une cupule 3 montée par l'intermédiaire d'une entretoise 2.

La platine-support 1 qui, de préférence est réalisée en acier inoxydable ou en Chrome-Cobalt présentant ou non un revêtement bicouche microporeux titane et hydroxyapatite, constitue le socle du cotyle prothétique.

Elle permet de répondre aux exigences anatomiques et mécaniques de l'articulation de la hanche.

Elle se présente essentiellement sous la forme d'un anneau de soutien 10 destiné à loger partiellement l'entretoise 2 et la cupule 3, et qui est muni de pattes internes 11 percées d'un oeillet 12 de forme ronde permettant le passage de vis d'ancrage 4 qui seront décrites plus loin. On notera que les pattes internes 11 peuvent comporter plusieurs oeillets 12, lesquels peuvent également présenter une forme oblongues.

La platine-support 1 comporte également, de manière connue, deux pattes externes sus-acétabulaires 13 dans lesquelles sont pratiquées des oeillets 14 pour le passage de vis, non représentées, pour la fixation sur le delta iliaque, ainsi qu'un crochet 15 permettant un appui externe dans l'échancrure ischiopubienne.

L'entretoise 2 se présente sous la forme d'une bague 20 qui comporte extérieurement une zone distale 21 de profil conique en sorte de permettre un assemblage de type à cônes morses avec l'anneau de soutien 10 qui présente intérieurement une zone proximale 16 de profil complémentaire. Elle comporte de plus extérieurement dans une zone sensiblement médiane, une collerette périphérique 22 percée d'une multiplicité d'oeillets 23, et destinée à être accolée à une collerette 17 que comporte la platine-support 1 en bordure de son ouverture, et qui est percée de trous taraudés 18 susceptibles d'être mis en regard d'oeillets 23 et de recevoir des vis 5, en vue d'une part de l'immobilisation en rotation de l'entretoise 2 sur la platine-support 1 après avoir choisi une orientation, et d'autre part du verrouillage de cet assemblage.

L'entretoise 2 est de forme générale en coin, c'est-à-dire que les plans de jonction avec respectivement la platine-support 1 et la cupule 3 sont convergents, en sorte d'autoriser, par le choix de ses caractéristiques, une orientation angulaire permettant de recentrer le point d'appui vertical anatomique à l'intérieur de la cupule 3 et d'augmenter par conséquent la congruence et la taille de la surface portante.

La cupule 3 est de forme générale hémisphérique avec un léger débord 30 en forme de casquette, elle comporte une cavité hémisphérique 31 destinée à recevoir un insert 6, non représenté sur la figure 1 mais visible sur la figure 2, comportant lui-même une cavité hémisphérique 60 destinée à recevoir la tête sphérique d'un élément fémoral prothétique.

L'assemblage de la cupule 3 est réalisé par un système de type à cônes morses, l'entretoise 2 comporte ainsi une zone conique interne 24, tandis que la cupule 3 comporte une zone conique externe 33. Par ailleurs, la cupule 3 comporte extérieurement un léger rebord 32 équatorial constituant un élément de déverrouillage du cône morse pour son extraction.

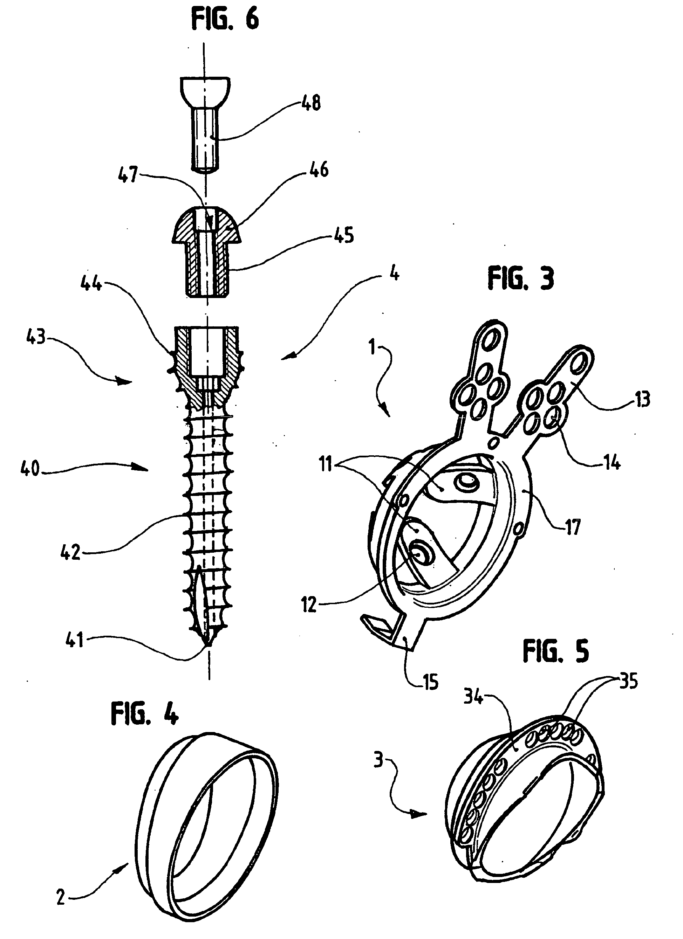

Le cotyle prothétique selon l'invention peut présenter plusieurs variantes, ainsi, en référence à la figure 2 et aux figures 3, 4 et 5, on peut voir un autre cotyle prothétique selon l'invention, constitué également de l'assemblage d'une platine-support 1, d'une entretoise 2 et d'une cupule 3, où ces différents éléments présentent chacun une construction différente.

Sur les figures 2 et 3 on peut ainsi constater que la platine-support 1 présente des pattes internes 11 d'une autre forme, notamment plus longues.

Sur les figures 2 et 4 on peut voir une entretoise 2 qui ne comporte pas de collerette.

Et sur les figures 2 et 5 on peut voir une cupule 3 qui comporte extérieurement une collerette 34 percée d'une multiplicité d'oeillets 35.

En référence maintenant à la figure 6, on peut voir une vis d'ancrage 4 qui permet de réaliser le réglage du positionnement de la platine-support 1 dans la cavité osseuse.

Cette vis d'ancrage 4, qui de préférence est canulée pour faciliter sa mise en place, comprend une partie distale 40 filetée présentant une pointe 41 et un filetage 42 de type spongieux, et une partie proximale 43 présentant la forme d'un cylindre percé axialement d'un canal taraudé 44 où peut être vissé un piston fileté 45, lui-même muni d'une tête 46 et percé d'un puits taraudé 47 destiné à recevoir une contre-vis de verrouillage 48.

Le piston fileté 45 permet, selon sa profondeur d'enfoncement dans le canal taraudé 44, de régler la hauteur des surfaces d'appui de la platine-support 1, tandis que la contre-vis 48 assure le maintien de ladite platine-support 1.

On notera que, de préférence, les différents filetages sont de pas inverses.

Après ancrage de la partie distale dans la matière osseuse, le vissage ou le dévissage du piston 45 permet de régler la hauteur de la tête 46 qui est destinée à servir d'appui à la platine-support 1 et plus exactement à une patte acétabulaire 11. Après détermination de la longueur adéquate d'enfoncement du piston 45 dans le canal 44, la platine-support 1 est solidarisée au moyen de la contre-vis de verrouillage 48 serrée dans le puits 47.

Bien entendu la partie proximale 43, le piston 47 et la contre-vis 48 sont munis chacun d'une empreinte de manoeuvrement de type empreinte creuse axiale.

On notera que les vis 5, les contre-vis de verrouillage 48, ainsi que les vis, non représentées, de fixation des pattes externes sus-acétabulaires 13, présentent des têtes dont la surface d'appui présente un profil courbe, de type hémisphérique, tandis que les éléments, respectivement 22, 34, 11 et 13, dans lesquels sont réalisés les oeillets, respectivement 23, 35, 12 et 14, présentent au niveau de ces derniers un fraisage de profil complémentaire apte à autoriser l'orientation de ces vis, par rapport aux éléments 22, 34, 11 et 13, selon l'axe désiré.

D'autre part, on notera qu'avantageusement la base de la partie proximale 43, c'est-à-dire la zone de liaison avec la partie distale 40, est tronconique, et le filetage 42 s'y prolonge, en sorte d'assurer un ancrage sûr.

Le cotyle prothétique modulaire selon l'invention est particulièrement destiné au traitement chirurgical de la reconstruction de l'articulation de la hanche dans les cas de reprises totales de prothèses de hanche, notamment lors de grandes destructions osseuses, et d'autre part dans les cas de dysplasies.

On notera que la vis d'ancrage 4 n'est pas d'un usage limité à la fixation d'un cotyle prothétique modulaire selon l'invention, elle peut trouver une autre application en orthopédie.

## Revendications

1. Cotyle prothétique modulaire pour le traitement chirurgical de la reconstruction de l'articulation de la hanche dans les cas de reprises totales de prothèses de hanche, comportant d'une part une platine-support (1) destinée à être disposée dans la cavité acétabulaire et ancrée au moyen de vis d'ancrage (4), et d'autre part une cupule métallique (3) destinée à être assemblée à ladite platine support (1), le cotyle prothétique étant **caractérisé en ce qu'**il comprend une entretoise orientable (2) verrouillée sur ladite platine-support (1) par un système d'assemblage de type à cônes morses (16, 21); tandis que ladite cupule métallique (3) est assemblée à ladite entretoise (2) par un système d'assemblage de type à cônes morses (24, 33).

2. Cotyle prothétique modulaire selon la revendication 1, **caractérisé en ce que** la platine-support (1) comporte au moins une patte d'appui interne acétabulaire (11) munie d'oeillets (12) permettant un ancrage intra-acétabulaire de ladite platine-support (1) au moyen des vis d'ancrage (4).

3. Cotyle prothétique modulaire selon la revendication 1 ou la revendication 2, **caractérisé en ce que** la platine-support (1) comporte au moins une patte externe sus-acétabulaire (13) munie d'oeillets (14) destinés à recevoir des vis de fixation de type spongieux ou cortical, ainsi qu'un crochet obturateur (15) apte à assurer un crochetage sous-acétabulaire.

4. Cotyle prothétique modulaire selon la revendication 2 ou la revendication 3, **caractérisé en ce que** les pattes (11, 13) présentent au niveau de chacun des oeillets (12, 14) qu'elles comportent, un profil concave apte, en coopération avec les vis (4) destinées à être introduites dans lesdits oeillets (12, 14) et dont les têtes présentent une surface d'appui de profil hémisphérique, à permettre une orientation desdites vis (4) selon plusieurs axes.

5. Cotyle prothétique modulaire selon l'une quelconque des revendications précédentes, **caractérisé en ce qu'**il comporte un revêtement ostéoconducteur bicouche titane et hydroxyapatite.

6. Cotyle prothétique modulaire selon l'une quelconque des revendications précédentes, **caractérisé en ce que** l'entretoise (2) présente une forme de coin, et elle comporte d'une part un cône mâle (21) situé à sa base et venant se verrouiller sur un cône femelle (16) que comporte de la platine-support (1); et d'autre part un cône femelle (24) situé sur sa partie supérieure, qui lui permet d'accueillir le cône mâle (33) que comporte la cupule (3), ce qui autorise l'orientation de cette dernière selon les besoins, quelle que soit la position de ladite platine-support (1).

7. Cotyle prothétique modulaire selon la revendication 6, **caractérisé en ce que** l'entretoise (2) est pourvue, sur sa partie extérieure, au centre de sa partie la plus large, et sur plus de la moitié de sa circonférence, d'une collerette de verrouillage (22) comprenant en divers points des oeillets (23) permettant le passage de vis (5) destinées à être serrées dans des trous taraudés (18) pratiqués dans un épaulement périphérique (17) que comporte la platine-support (1), ladite collerette (22) présentant au niveau de chacun desdits oeillets (23) un profil concave apte, en coopération avec lesdites vis (5) dont les têtes présentent une surface d'appui de profil hémisphérique, à permettre une orientation desdites vis (5) selon plusieurs axes.

8. Cotyle prothétique modulaire selon la revendication 6, **caractérisé en ce que** la cupule (3) est munie sur sa zone équatoriale externe, sur plus de la moitié de sa circonférence, et au-dessus de son cône mâle, d'une collerette (34) apte à permettre un verrouillage supplémentaire de la platine-support (1), de l'entretoise (2) et de la cupule (3), à cet effet ladite collerette (34) comprend en divers points des oeillets (35) permettant le passage de vis (5) destinées à être serrées dans des trous taraudés (18) pratiqués dans un épaulement périphérique (17) que comporte la platine-support (1), ladite collerette (34) présentant au niveau de chacun desdits oeillets (35) un profil concave apte, en coopération avec lesdites vis (5) dont les têtes présentent une surface d'appui de profil hémisphérique, à permettre une orientation desdites vis (5) selon plusieurs axes.

9. Cotyle prothétique modulaire selon l'une quelconque des revendications précédentes, **caractérisé en ce que** la cupule (3) présente une partie interne (31) totalement borgne, de type miroir, qui permet la libre mobilité d'un insert polymère (6) qu'elle y accueille, lequel insert (6) présente une cavité hémisphérique (60) destinée à recevoir la tête sphérique d'un élément fémoral prothétique.

10. Cotyle prothétique modulaire selon l'une quelconque des revendications 1 à 8, **caractérisé en ce que** la cupule (3) présente une partie interne totalement borgne présentant une partie femelle et destinée à accueillir un insert céramique présentant un profil conique mâle en vue de réaliser un assemblage de type à cônes morses, ledit insert comportant une cavité hémisphérique destinée à recevoir la tête sphérique d'un élément fémoral prothétique.

11. Cotyle prothétique modulaire selon l'une quelconque des revendications précédentes, **caractérisé en ce que** les vis d'ancrage (4) comportent des moyens (44, 45, 48) aptes à assurer le réglage affiné de la hauteur des surfaces d'appui dudit cotyle, et l'ancrage de ce dernier dans le tissu osseux tout en assurant un réglage équilibré des forces de pression.

12. Cotyle prothétique modulaire selon la revendication 11, **caractérisé en ce que** les vis d'ancrage (4) comportent un corps comprenant une partie proximale (43) de forme cylindrique prolongée d'une partie distale (40) terminée en pointe et présentant un filetage (42) de type spongieux auto taraudeur, ladite partie proximale étant percée axialement d'un canal taraudé (44) destiné à accueillir un piston fileté (45), lui-même muni d'une tête (46) et percé d'un puits taraudé (47) destiné à recevoir une contre-vis de verrouillage (48).

13. Cotyle prothétique modulaire selon la revendication 12, **caractérisé en ce que** la contre-vis (48) comporte une tête dont la face d'appui présente un profil courbe de type hémisphérique, destiné à coopérer avec l'élément à fixer (22, 34, 11, 13) qui présente, au niveau de l'oeillet (23, 35, 12, 14) destiné à recevoir ladite contre-vis (48), un profil complémentaire, en sorte d'autoriser l'orientation de ladite vis (48), par rapport audit élément (22, 34, 11, 13), selon l'axe désiré.

## Claims

1. Modular acetabular cup for surgical treatment of the reconstruction of the hip joint in case of complete reparations of hip prostheses, including on the one hand a support plate (1) designed to be placed in the acetabular cavity and anchored by means of anchoring screws (4), and on the other hand a metal cup (3) designed to be assembled to said support plate (1), the acetabular cup being **characterized in that** it includes an adjustable spacer (2) locked on said support plate (1) by an assembly system of the type having conical jaws (16, 21); whereas said metal cup (3) is assembled to said spacer (2) by an assembly system of the type having conical jaws (24, 33).

2. Modular acetabular cup according to claim 1, **characterized in that** the support plate (1) includes at least an internal acetabular bearing arm (11) provided with eyelets (12) permitting intra-acetabular anchoring of said support plate (1) by means of anchoring screws (4).

3. Modular acetabular cup according to claim 1 or claim 2, **characterized in that** the support plate (1) includes at least an external super-acetabular arm (13) provided with eyelets (14) designed to receive cortical or sponge-like fastening screws, as well as an obturating hook (15) capable of ensuring a sub-acetabular hooking.

4. Modular acetabular cup according to claim 2 or claim 3, **characterized in that** the arms (11, 13) have at the level of each eyelet (12, 14) that they include, a concave profile capable, in cooperation with the screws (4) designed to be introduced in said eyelets (12, 14) and whose heads have a bearing surface of a hemispheric profile, of permitting orientation of said screws (4) according to several axes.

5. Modular acetabular cup according to any of the preceding claims, **characterized in that** it includes a double-layer osteoconductive coating of titanium and hydroxyapatite.

6. Modular acetabular cup according to any of the preceding claims, **characterized in that** the spacer (2) has the shape of a wedge, and it includes on the one hand an external cone (21) situated at its base and being locked on an internal cone (16) the support plate (1) includes; and on the other hand an internal cone (24) situated on its superior portion, permitting it to receive the external cone (33) the cup (3) includes, which allows adjustment of the latter according to the needs, whatever the position of said support plate (1).

7. Modular acetabular cup according to claim 6, **characterized in that** the spacer (2) is provided, on its external portion, at the center of its widest portion, and over more than half of its circumference, with a locking flange (22) including, at various spots, eyelets (23) permitting the passage of screws (5) designed to be tightened in tapped holes (18) made in a peripheral shoulder (17) the support plate (1) includes, said flange (22) having, at the level of each eyelet (23) a concave profile capable, in cooperation with said screws (5) whose heads have a bearing surface of a hemispheric profile, of permitting orientation of said screws (5) according to several axes.

8. Modular acetabular cup according to claim 6, **characterized in that** the cup (3) is provided on its external equatorial zone, over more than half of its circumference, and above its external cone, with a flange (34) capable of permitting supplemental locking of the support plate (1), of the spacer (2) and of the cup (3), for this purpose said flange (34) includes, at various spots, eyelets (35) permitting the passing through of screws (5) designed to be tightened in tapped holes (18) made in a peripheral shoulder (17) the support plate (1) includes, said flange (34) having, at the level of each eyelet (35) a concave profile capable, in cooperation with said screws (5) whose heads have a bearing surface of a hemispheric profile, of permitting orientation of said screws (5) according to several axes.

9. Modular acetabular cup according to any of the preceding claims, **characterized in that** the cup (3) has a totally obstructed mirror-like internal portion (31), permitting free mobility of a polymeric insert (6) that it receives therein, which insert (6) has a hemispheric cavity (60) designed to receive the spherical head of a prosthetic femoral component.

10. Modular acetabular cup according to any of the claims 1 through 8, **characterized in that** the cup (3) has a totally obstructed internal portion having a female portion and designed to receive a ceramic insert having a male conical profile in view of performing an assembly of the type having conical jaws, said insert including a hemispheric cavity designed to receive the spherical head of a prosthetic femoral component.

11. Modular acetabular cup according to any of the preceding claims, **characterized in that** the anchoring screws (4) include means (44, 45, 48) capable of ensuring the fine adjustment of the height of bearing surfaces of said acetabular cup, and the anchoring of the latter in the bone tissue yet ensuring a balanced adjustment of pressure forces.

12. Modular acetabular cup according to claim 11, **characterized in that** the anchoring screws (4) include a body comprising a cylindrical proximal portion (43) extended by a distal portion (40) ending with a point and having a thread (42) of the self-cutting spongy type, said proximal portion being bored axially by a tapped channel (44) designed to receive a threaded piston (45), itself provided with a head (46) and bored by a tapped well (47) designed to receive a locking counter screw (48).

13. Modular acetabular cup according to claim 12, **characterized in that** the counter screw (48) includes a head whose bearing face has a bended hemispheric profile, designed to co-operate with the component to be fixed (22, 34, 11, 13) which has, at the level of the eyelet (23, 35, 12, 14) designed to receive said counter screw (48), a complementary profile, in order to permit the adjustment of said screw (48), with respect to said component (22, 34, 11, 13), according to the desired axis.

## Patentansprüche

1. Modulare prothetische Gelenkpfanne für die chirurgische Behandlung bei der Rekonstruktion des Hüftgelenks in den Fällen von Hüftprothesen-Totalwechsel, umfassend, einerseits, eine Trägerplatte (1), vorgesehen, um in den acetabularen Hohlraum angeordnet und mit Hilfe von Verankerungsschrauben (4) verankert zu werden, und, andererseits, eine Schutzhaube aus Metall (3), vorgesehen, um mit der besagten Trägerplatte (1) verbunden zu werden, wobei die prothetische Gelenkpfanne **dadurch gekennzeichnet** sei, daß sie ein richtbares Zwischenstück (2) umfaßt, das an der besagten Trägerplatte (1) durch ein System zum Zusammenbau der Art mit Morsekegeln (16, 21) verriegelt ist, während die besagte Schutzhaube aus Metall (3) mit dem besagten Zwischenstück (2) durch ein System zum Zusammenbau der Art mit Morsekegeln (24, 33) verbunden ist.

2. Modulare prothetische Gelenkpfanne nach Anspruch 1, **dadurch gekennzeichnet, daß** die Trägerplatte (1) zumindest eine innere acetabulare Stützöse (11) umfaßt, die mit Verankerungslöchern (12) ausgestattet ist, erlaubend eine intraacetabulare Verankerung der besagten Trägerplatte (1) mit Hilfe der Verankerungsschrauben (4).

3. Modulare prothetische Gelenkpfanne nach Anspruch 1 oder Anspruch 2, **dadurch gekennzeichnet, daß** die Trägerplatte (1) zumindest eine äußere superacetabulare Öse (13), ausgestattet mit Verankerungslöchern (14), die vorgesehen sind, um Befestigungsschrauben der Art spongiös oder kortikal aufzunehmen, sowie einen verschließenden Haken (15), geeignet, um eine subacetabulare Befestigung zu sichern, umfaßt.

4. Modulare prothetische Gelenkpfanne nach Anspruch 2 oder Anspruch 3, **dadurch gekennzeichnet, daß** die Ösen (11, 13) im Bereich von jedem der Verankerungslöcher (12, 14), die sie umfassen, ein konkaves Profil aufweisen, geeignet, um, in Zusammenwirkung mit den Schrauben (4), die vorgesehen sind, um in die besagten Verankerungslöcher (12, 14) eingeführt zu werden und deren Köpfe eine Stützoberfläche mit halbkugelförmigem Profil aufweisen, geeignet, um eine Orientierung der besagten Schrauben (4) nach mehreren Achsen zu erlauben.

5. Modulare prothetische Gelenkpfanne nach irgendeinem der vorgehenden Ansprüche, **dadurch gekennzeichnet, daß** sie eine zweilagige osteokonduktive Beschichtung aus Titan und Hydroxyapatit umfaßt.

6. Modulare prothetische Gelenkpfanne nach irgendeinem der vorgehenden Ansprüche, **dadurch gekennzeichnet, daß** das Zwischenstück (2) eine Keilform aufweist, und es umfaßt, einerseits, einen männlichen Konus (21), der an seinem unteren Teil befindlich ist und sich an einem weiblichen Konus (16) verriegelt, den die Trägerplatte (1) umfaßt, und, andererseits, einen weiblichen Konus (24), der an seinem oberen Teil befindlich ist, der ihm erlaubt, den männlichen Konus (33), den die Schutzhaube (3) umfaßt, aufzunehmen, was die Orientierung dieser Letzteren je nach Bedarf, ungeachtet der Position der besagten Trägerplatte (1), erlaubt.

7. Modulare prothetische Gelenkpfanne nach Anspruch 6, **dadurch gekennzeichnet, daß** das Zwischenstück (2) auf seinem Außenteil, im Zentrum von seinem breitesten Teil und auf mehr als der Hälfte seines Umfangs, mit einem Verriegelungskragen (22) ausgestattet ist, umfassend an verschiedenen Stellen Verankerungslöcher (23), erlaubend den Durchgang der Schrauben (5), die vorgesehen sind, um in Gewindelöcher (18) gespannt zu werden, die in einem peripheren Flansch (17) vorgesehen sind, den die Trägerplatte (1) umfaßt, wobei der besagte Kragen (22) im Bereich von jedem der besagten Verankerungslöcher (23) ein konkaves Profil aufweist, geeignet, um, in Zusammenwirken mit den besagten Schrauben (5), deren Köpfe eine Stützoberfläche mit halbkugelförmigem Profil aufweisen, eine Orientierung der besagten Schrauben (5) nach mehreren Achsen zu erlauben.

8. Modulare prothetische Gelenkpfanne nach Anspruch 6, **dadurch gekennzeichnet, daß** die Schutzhaube (3) an ihrem äußeren äquatorialen Bereich, auf mehr als der Hälfte ihres Umfangs und über ihrem männlichen Konus, mit einem Kragen (34) ausgestattet ist, der geeignet ist, um eine zusätzliche Verriegelung der Trägerplatte (1), des Zwischenstücks (2) und der Schutzhaube (3) zu erlauben, wobei der besagte Kragen (34) zu diesem Zweck an verschiedenen Stellen Verankerungslöcher (35) umfaßt, erlaubend den Durchgang der Schrauben (5), die vorgesehen sind, um in Gewindelöcher (18) gespannt zu werden, die in einem peripheren Flansch (17) vorgesehen sind, den die besagte Trägerplatte (1) umfaßt, wobei der besagte Kragen (34) im Bereich von jedem der besagten Verankerungslöcher (35) ein konkaves Profil aufweist, geeignet, um, in Zusammenwirkung mit den besagten Schrauben (5), deren Köpfe eine Stützoberfläche mit halbkugelförmigem Profil aufweisen, eine Orientierung der besagten Schrauben (5) nach mehreren Achsen zu erlauben.

9. Modulare prothetische Gelenkpfanne nach irgendeinem der vorgehenden Ansprüche, **dadurch gekennzeichnet, daß** die Schutzhaube (3) einen gänzlich blinden, einseitig offenen inneren Spiegelteil (31) aufweist, der die freie Beweglichkeit eines polymeren Einsatzes (6) erlaubt, den sie dort aufnimmt, wobei dieser Einsatz (6) einen halbkugelförmigen Hohlraum (60) aufweist, vorgesehen, um den sphärischen Kopf eines prothetischen femoralen Elements aufzunehmen.

10. Modulare prothetische Gelenkpfanne nach irgendeinem der Ansprüche 1 bis 8, **dadurch gekennzeichnet, daß** die Schutzhaube (3) einen gänzlich blinden, einseitig offenen inneren Teil aufweist, aufweisend einen weiblichen Teil, und vorgesehen, um einen Keramikeinsatz aufzunehmen, der ein männliches konisches Profil aufweist, um eine Zusammenfügung der Art mit Morsekegeln zu schaffen, wobei der besagte Einsatz einen halbkugelförmigen Hohlraum umfaßt, vorgesehen, um den sphärischen Kopf eines prothetischen femoralen Elements aufzunehmen.

11. Modulare prothetische Gelenkpfanne nach irgendeinem der vorgehenden Ansprüche, **dadurch gekennzeichnet, daß** die Verankerungsschrauben (4) Mittel (44, 45, 48) umfassen, die geeignet sind, um die feine Einstellung der Höhe der Stützoberflächen der besagten Gelenkpfanne und die Verankerung dieser Letzteren im Knochenstoff zu sichern, indem eine ausgeglichene Einstellung der Druckkräfte gesichert wird.

12. Modulare prothetische Gelenkpfanne nach Anspruch 11, **dadurch gekennzeichnet, daß** die Verankerungsschrauben (4) einen Körper umfassen, umfassend einen proximalen Teil (43) zylindrischer Form, verlängert durch einen distalen Teil (40), spitz zulaufend und aufweisend ein selbstschneidendes Gewinde (42) der Art spongiös, wobei der besagte proximale Teil axial von einem Gewindekanal (44) durchstochen sei, vorgesehen, um einen Gewindekolben (45) aufzunehmen, der selbst mit einem Kopf (46) ausgestattet ist und von einer Gewindesenke (47) durchstochen wird, vorgesehen, um eine Gegenschraube zur Verriegelung (48) aufzunehmen.

13. Modulare prothetische Gelenkpfanne nach Anspruch 12, **dadurch gekennzeichnet, daß** die Gegenschraube (48) einen Kopf umfaßt, dessen Stützoberfläche ein gebogenes Profil der Art Halbkugel aufweist, vorgesehen, um mit dem zu befestigenden Element (22, 34, 11, 13) zusammenzuwirken, das im Bereich des Verankerungslochs (23, 35, 12, 14), vorgesehen, um die besagte Gegenschraube (48) aufzunehmen, ein ergänzendes Profil aufweist, derart, um die Orientierung der besagten Schraube (43) hinsichtlich des besagten Elements (22, 34, 11, 13) nach der gewünschten Achse zu erlauben.
